(19) Europäisches Patentamt
European Patent Office
Office européen des brevets

(11) EP 1 245 557 B1

(12) # EUROPÄISCHE PATENTSCHRIFT

(45) Veröffentlichungstag und Bekanntmachung des Hinweises auf die Patenterteilung:
**21.07.2004 Patentblatt 2004/30**

(51) Int Cl.[7]: **C07C 69/52**, C07C 69/54, C09D 4/00

(21) Anmeldenummer: **02006134.7**

(22) Anmeldetag: **19.03.2002**

(54) **Giessbare härtbare Fett(meth)acrylate**

Castable curable fatty acid (meth)acrylates

Coulable et réticulable (méth)acrylates d'acides gras

(84) Benannte Vertragsstaaten:
**AT BE CH CY DE DK ES FI FR GB GR IE IT LI LU MC NL PT SE TR**

(30) Priorität: **28.03.2001 DE 10115245**

(43) Veröffentlichungstag der Anmeldung:
**02.10.2002 Patentblatt 2002/40**

(73) Patentinhaber: **Cognis Deutschland GmbH & Co. KG**
**40589 Düsseldorf (DE)**

(72) Erfinder:
- **Westfechtel, Alfred, Dr.**
  **40724 Hilden (DE)**
- **Sulzbach, Horst, Dr.**
  **27721 Ritterhude (DE)**
- **Zander, Lars, Dr.**
  **40597 Düsseldorf (DE)**

(56) Entgegenhaltungen:
**FR-A- 2 561 249**
**US-A- 3 125 592**
**US-A- 3 979 270**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 245 557 B1

## Beschreibung

### Gebiet der Erfindung

Die vorliegende Erfindung betrifft gießbare härtbare Fett(meth)acrylate sowie deren Verwendung zur Herstellung von Beschichtungen.

### Stand der Technik

Der Einsatz der Strahlungshärtung in der Beschichtungsindustrie zur Erzeugung hochwertiger Beschichtungsmaterialien ist aus dem Stand der Technik bekannt. Hier werden olefinisch ungesättigte Verbindungen (Monomere, Oligomere, Polymere, Prepolymere), also Verbindungen, die als Strukturelemente C=C-Doppelbindungen enthalten, durch Einwirkung energiereicher Strahlung, beispielsweise UV-Licht oder Elektronenstrahlung, ausgehärtet. Gelegentlich wird vor dieser eigentlichen Strahlungshärtung auch eine physikalische Trocknung vorgenommen.

**US-A-3,979,270** beschreibt in Spalte 2, Zeilen 4 bis 21, die Umsetzung von epoxidiertem Sojaöl mit Acryl- bzw. Methacrylsäure. Die dabei erhaltenen Produkte, die man als Fett(Meth)acrylate bezeichnen kann, sind einer anschließenden Aushärtung zu Polymeren zugänglich, die auf unterschiedliche Weise realisiert werden kann. Ein Nachteil der aus dem Stande der Technik bekannten Fett(Meth)acrylate besteht darin, daß mit zunehmender Jodzahl der diesen Verbindungen zu Grunde liegenden Fett bzw. Öle die Viskosität der Fett(meth)acrylate stark zunimmt.
So hat Sojaölacrylat eine für die Praxis genügend geringe Viskosität, um gut handhabbar zu sein, die aus Sojaölacrylat entstehenden Polymerisate weisen jedoch nur mäßige Härtegrade auf. Andererseits hat polymerisiertes Leinölacrylat die erwünscht hohen Härtegrade, allerdings besitzt Leinölacrylat eine äußerst hohe Viskosität, die für die Praxis erheblich zu groß ist, um die Substanz handhaben zu können.

### Beschreibung der Erfindung

Aufgabe der vorliegenden Erfindung war die Bereitstellung gießbarer härtbarer Substanzen. Diese Substanzen sollten einerseits eine Viskosität von weniger als 30.000 mPas und vorzugsweise von weniger als 20.000 mPas (gemessen in Substanz nach Brookfield bei 23 °C) aufweisen und gleichzeitig sich dadurch auszeichnen, daß sie bei der radikalisch initiierten Härtung (durchgeführt in Gegenwart von 5 Gew.-% - bezogen auf die Substanz - tert.-Butylperoxidbenzoat bei 160 °C in einem Zeitraum von 30 Minuten) zu einem Polymeren mit einer Shore-D-Härte (bestimmt bei 23 °C nach DIN 53505) von mindestens 60 führen. Diese beiden gleichzeitig zu erfüllenden Bedingungen bedeuten, daß die Substanzen einerseits gut handhabbar, d.h. auf Grund ihrer relativ geringen Viskosität insbesondere gießbar und/oder pumpbar sein sollen, andererseits bei der radikalisch initiierten Aushärtung zu Polymeren mit der erwünschten hohen Härte (Shore-D-Werte von mindestens 60 bei 23 °C) führen.

Die genannte Aufgabe wurde überraschenderweise gelöst durch Fett(meth)acrylate, erhältlich durch Umsetzung von epoxidierten Fettsäurestern und/oder epoxidierten Triglyceriden mit Acrylsäure und/oder Methacrylsäure, wobei die Umsetzung so geführt wird, daß der %-EpO-Gehalt der Fett(meth)acrylate unterhalb von 0,6 liegt und wobei für die den Fett(meth)acrylaten zu Grunde liegenden epoxidierten Fettsäurester und/oder epoxidierten Triglyceride folgende Maßgaben gelten:

a) Der %-EpO-Gehalt der eingesetzten epoxidierten Fettsäurestern und/oder epoxidierten Triglyceride liegt oberhalb von 6,4.
b) Weniger als 20 mol-% der den epoxidierten Fettsäurestern und/oder epoxidierten Triglyceriden zu Grunde liegenden Fettsäurebausteine enthalten 3 oder mehr C=C-Doppelbindungen pro Fettsäurebaustein.
c) Mehr als 60 mol-% der den epoxidierten Fettsäurestern und/oder epoxidierten Triglyceriden zu Grunde liegenden Fettsäurebausteine enthalten 2 C=C-Doppelbindungen pro Fettsäurebaustein.

Unter Fett(meth)acrylaten sind sowohl Fettacrylate als auch Fettmethacrylate zu verstehen. Fettacrylate und Fettmethacrylate können im Rahmen der vorliegenden Erfindung sowohl als Einzelsubstanzen, als auch im Gemisch untereinander eingesetzt werden. Dabei können sowohl Gemische von Fettacrylaten, als auch Gemische von Fettmethacrylaten eingesetzt werden. Ebenso können Gemische eingesetzt werden, die sowohl Fettacrylate als auch Fettmethacrylate enthalten.

<u>Zu Maßgabe a)</u>

Die dem Fachmann bekannte Epoxidzahl (%EpO-Gehalt) gibt an, wieviel Gramm Oxiran-Sauerstoff in 100 Gramm einer Probe enthalten sind. Die Epoxidzahl wird durch Titration ermittelt. Hierzu sei ausdrücklich auf den Beispielteil verwiesen, wo die im Rahmen dieser Erfindung zu benutzende Meßmethode ausführlich dargestellt ist.
In einer Ausführungsform liegt der %-EpO-Gehalt der eingesetzten epoxidierten Fettsäurestern und/oder epoxidierten Triglyceride oberhalb von 7,0.

Zu Maßgabe b)

In einer Ausführungsform enthalten weniger als 10 mol-% der den epoxidierten Fettsäurestern und/oder epoxidierten Triglyceriden zu Grunde liegenden Fettsäurebausteine 3 oder mehr C=C-Doppelbindungen pro Fettsäurebaustein.
In einer bevorzugten Ausführungsform enthalten weniger als 5 mol-% der den epoxidierten Fettsäurestern und/oder epoxidierten Triglyceriden zu Grunde liegenden Fettsäurebausteine 3 oder mehr C=C-Doppelbindungen pro Fettsäurebaustein.

Zu Maßgabe c)

In einer Ausführungssform enthalten mehr als 65 mol-% der den epoxidierten Fettsäurestem und/oder epoxidierten Triglyceriden zu Grunde liegenden Fettsäurebausteine 2 C=C-Doppelbindungen pro Fettsäurebaustein.
In einer bevorzugten Ausführungssform enthalten mehr als 70 mol-% der den epoxidierten Fettsäurestern und/oder epoxidierten Triglyceriden zu Grunde liegenden Fettsäurebausteine 2 C=C-Doppelbindungen pro Fettsäurebaustein.
Eine optionale Ausführungssform der Erfindung ist dadurch gekennzeichnet, daß weniger als 20 mol-% - vorzugsweise weniger als 10 mol-% - der den epoxidierten Fettsäurestern und/oder epoxidierten Triglyceriden zu Grunde liegenden Fettsäurebausteine frei von C=C-Doppelbindungen sind, in dem Sinne, daß es sich bei diesen Fettsäurebausteinen um gesättigte Fettsäuren handelt.

Zur Herstellung der Fett(meth)acrylate

Wie bereits ausgeführt bezieht sich die Erfindung in einer ersten Ausführungsform auf Fett(meth)acrylate, die erhältlich sind durch Umsetzung von epoxidierten Fettsäurestern und/oder epoxidierten Triglyceriden mit Acrylsäure und/oder Methacrylsäure. Dabei soll der %-EpO-Gehalt der Fett(meth)acrylate auf Werte unterhalb von 0,6 eingestellt werden.
Sofern zur Herstellung der erfindungsgemäßen Fett(meth)acrylate epoxidierte Fettsäurester und/oder epoxidierte Triglyceride mit Acrylsäure und/oder Methacrylsäure umgesetzt werden, bedeutet die genannte Bedingung, daß die in den Ausgangsstoffen, nämlich den epoxidierten Fettsäurestern und/oder epoxidierten Triglyceriden vorhandenen Oxiranringe weitgehend ringgeöffnet werden. Vorzugsweise führt man die Oxiran-Ringöffnung nahezu quantitativ durch.
Die Herstellung epoxidierter Fettsäurester bzw. epoxidierter Triglyceride ist seit langem bekannt. Hierzu werden Ester von olefinisch ungesättigten Fettsäuren bzw. Triglyceriden, die als Fettsäurebausteine olefinisch ungesättigte Fettsäuren enthalten, der Epoxidation unterworfen, wobei eine oder mehrere Doppelbindungen pro Molekül in Oxirangruppen überführt werden.
Als Fettsäurebausteine der zu epoxidierenden Fettsäureester sind Carbonsäuren mit 12 bis 24 C-Atomen, die mindestens eine olefinische Doppelbindung im Molekül enthalten, bevorzugt. Bei den zu epoxidierenden Triglyceriden sind solche Triglyceride bevorzugt, bei denen mindestens ein Fettsäurebaustein pro Triglyceridmolekül mindestens eine olefinische Doppelbindung enthält.
Beispiele geeigneter epoxidierter Triglyceride sind die Epoxidationsprodukte folgender ungesättigter Öle: Linolaöl, Hanföl, Kugeldistelöl, Färberdistelöl, Sonnenblumenöl, Traubenkernöl, Mohnöl, Fichtensamenöl. Linolaöl ist hierbei ganz besonders bevorzugt.
Die Herstellung epoxidierter Triglyceride geschieht insbesondere durch Umsetzung der genannten ungesättigten Öle mit Perameisen- oder Peressigsäure.
Die Addition von Acryl- und/oder Methacrylsäure an die genannten epoxidierten Fettsäurester bzw. epoxidierten Triglyceride ist dem Fachmann an sich bekannt. Sie ist im Rahmen der vorliegenden Erfindung so durchzuführen, daß der %EpO-Gehalt der Fett(meth)acrylate unterhalb von 0,6 liegt.
Wie bereits gesagt ist es jedoch besonders bevorzugt, die Addition von Acryl- und/oder Methacrylsäure an die genannten epoxidierten Fettsäurester bzw. epoxidierten Triglyceride in der Weise durchzuführen, daß mehr oder weniger alle Oxiranringe geöffnet und in $HO-CH_2-CH_2-OR$-Gruppen überführt werden, in denen R einen Acrylat- bzw. Methacrylatrest bedeutet.

Verwendung zur Herstellung von Beschichtungsmassen

Die erfindungsgemäßen Fett(meth)acrylate eignen sich in hervorragender Weise zur Herstellung von Beschichtungsmassen, da es sich bei ihnen um härtbare Substanzen handelt.
Ein weiterer Gegenstand der Erfindung ist dementsprechend die Verwendung der erfindungsgemäßen Fett (meth)acrylate zur Herstellung von Beschichtungsmassen.
Die Aushärtung der erfindungsgemäßen Fett(meth)acrylate kann dabei sowohl radikalisch erfolgen, was auf Grund der in den Molekülen enthaltenen Doppelbindungen möglich ist. Die Aushärtung kann auch durch Umsetzung mit Isocyanaten erfolgen, da die Moleküle infolge der Oxiran-Ringöffnung freie OH-Gruppen aufweisen.
Es ist auch möglich, ein oder mehrere Fett(meth)acrylate einer 2-stufigen Behandlung zu unterwerfen, nämlich

- zunächst einer Umsetzung mit aliphatischen und/oder aromatischen Isocyanaten
- und anschließend einer radikalischen Nachvernetzung der dabei erhaltenen Polyurethane (pu*) in Gegenwart mindestens eines Radikalinitiators.

Insofern bei der Aushärtung der erfindungsgemäßen Fett(Meth)acrylate eine Umsetzung mit Isocyanaten erfolgt, unterliegt die Wahl der Isocyanate an sich keinen besonderen Einschränkungen. Prinzipiell lassen sich somit alle dem Fachmann einschlägig bekannten Isocyanate, also Verbindungen, die ein oder mehrere -N=C=O - Gruppen enthalten, einsetzen.
Vorzugsweise setzt man Diisocyanate, Oligo- bzw. Polyisocyanate, sowie Gemische dieser Verbindungen ein. Zu den Polyisocyanaten im Sinne der vorliegenden Erfindung gehören beispielsweise Addukte von Diisocyanaten an Trimethylolpropan, Biurete, Uretdione (cyclodimerisierte Isocyanate), Isocyanurate (cyclotrimerisierte Isocyanate), Allophanate, Carbodiimid-basierte Isocyanate und dergleichen.
Besonders hingewiesen sei auf handelsübliche Polyisocyanate, beispielsweise Polymer-MDI und dergleichen, die in verschiedenen Polymerisationsgraden kommerziell erhältlich sind.
Bei den Diisocyanaten setzt man vorzugsweise Verbindungen der allgemeinen Struktur O=C=N-X-N=C=O ein, wobei X ein aliphatischer, alicyclischer oder aromatischer Rest ist, vorzugsweise ein aliphatischer oder alicyclischer Rest mit 4 bis 18 C-Atomen.
Geeignete Diisocyanate sind beispielsweise: 1,5-Naphthylendiisocyanat, 4,4'-Diphenylmethandiisocyanat (= Methylen-diphenylen-diisocyanat, MDI), hydriertes MDI ($H_{12}$MDI, eine cyloaliphatische Verbindung), Xylylendiisocyanat (XDI), Tetramethylxylylendiisocyanat (TMXDI), 4,4'-Diphenyldimethylmethan-diisocyanat, Diund Tetraalkyldiphenylmethandiisocyanat, 4,4'-Dibenzyldiisocyanat, 1,3-Phenylendiisocyanat, 1,4-Phenylendiisocyanat, die Isomeren des Toluylendiisocyanats (TDI, insbesondere das technische Isomerengemisch aus im wesentlichen 2,4- und 2,6-Toluylendiisocyanat), 1-Methyl-2,4-diisocyanato-cyclohexan, 1,6-Diisocyanato-2,2,4-trimethylhexan, 1,6-Diisocyanato-2,4,4-trimethylhexan, 1-Isocyanatomethyl-3-isocyanato-1,5,5-trimethyl-cyclohexan (Isophorondiisocyanat = IPDI), chlorierte und bromierte Diisocyanate, phosphorhaltige Diisocyanate, 4,4'-Diisocyanatophenylperfluorethan, Tetramethoxybutan-1,4-diisocyanat, Butan-1,4-diisocyanat, Hexamethylendiisocyanat (HDI), Dicyclohexylmethandiisocyanat, Cyclohexan-1,4-diisocyanat, Ethylen-diisocyanat, Phthalsäure-bis-isocyanatoethylester, ferner Diisocyanate mit reaktionsfähigen Halogenatomen, wie 1-Chlormethylphenyl-2,4-diisocyanat, 1-Brommethylphenyl-2,6-diisocyanat, 3,3-Bis-chlormethylether-4,4'diphenyldiisocyanat. Schwefelhaltige Polyisocyanate erhält man beispielsweise durch Umsetzung von 2 mol Hexamethylen-diisocyanat mit 1 mol Thiodiglykol oder Dihydroxydihexylsulfid. Weitere wichtige Diisocyanate sind Trimethylhexamethylendiisocyanat, 1,4-Diisocyanatobutan, 1,12-Diisocyanatododecan und Dimerfettsäurediisocyanat (Handelsprodukt "Sovermol DDI 1410" der Cognis Deutschland GmbH).
Besonders geeignete Diisocyanate sind: Tetramethylen-, Hexamethylen-, Undecan-, Dodecamethylen-, 2,2,4-Trimethylhexan-, 1,3-Cyclohexan-, 1,4-Cyclohexan-, 1,3-bzw. 1,4-Tetramethylxylol-, Isophoron-, 4,4-Dicyclohexylmethan- und Lysinester-Diisocyanat.
In einer Ausführungsform der vorliegenden Erfindung setzt man höherfunktionelle Isocyanate ein, worunter solche Isocyanate verstanden werden, die eine mittlere NCO-Funktionalität von mindestens 2,0 aufweisen. Insbesondere sei hier auf alle handelsüblichen Polyisocyanate (beispielsweise Polymer-MDI und dergleichen) verwiesen, die eine NCO-Funktionalität oberhalb von 2,0 aufweisen. Wie dem Fachmann bekannt spricht man von mittlerer NCO-Funktionalität, weil die entsprechenden höherfunktionellen Isocyanate nicht zwingend in Form chemisch einheitlicher Individuen wie etwa cyclotrimerisierten Isocyanaten vorliegen müssen, sondern - insbesondere bei handelsüblichen technischen Produkten - häufig Gemische verschiedener chemischer Individuen darstellen, die jeweils definierte NCO-Funktionalitäten aufweisen.

Zusammensetzungen

Ein weiterer Erfindungsgegenstand betrifft gießbare, härtbare Zusammensetzungen mit einem Gehalt von einem oder mehreren Fett(meth)acrylaten. In einer Ausführungsform enthalten diese Zusammensetzungen neben den erfindungsgemäßen Fett(meth)acrylaten ein oder mehrere radikalisch copolymerisierbaren Verbindungen. Als derartige Verbindungen eignen sich dabei insbesondere Substanzen mit einer C=C-Doppelbindung pro Molekül, vorzugsweise Acrolein, Acrylamid, Vinylacetat und Styrol, wobei diese Verbindungen allein oder im Gemisch untereinander eingesetzt werden können.

In einer Ausführungsform werden die erfindungsgemäßen Fett(meth)acrylate werden - gewünschtenfalls in Kombination mit radikalisch copolymerisierbaren Verbindungen - in Gegenwart von bis zu 20 Gew.-% üblicher Kunststoffhilfsmittel der Aushärtung zu Beschichtungsmassen unterworfen (Gew.-% der Summe aller Kunststoffhilfsmittel bezogen auf die Gesamtmenge der eingesetzten Fett(meth)acrylate). Solche Kunststoffhilfsmittel sind beispielsweise Verdickungsmittel, Verlaufshilfsmittel, Entschäumer, Gleitmittel, Füllmittel, UV-Stabilisatoren. Solche Hilfsmittel sind dem Fachmann hinreichend aus der Lack- bzw. Beschichtungs-Technologie bekannt.

Als feste Substrate auf die die Beschichtungsmassen gemäß der vorliegenden Erfindung aufgebracht werden können, eignen sich insbesondere Holz, Papier, Kunststoffoberflächen, mineralische Baustoffe wie Zement-Formsteine oder Zementfaserplatten, ferner Metalle oder beschichtete Metalle.

Die Beschichtungsmassen werden in bekannter Weise auf das gewünschte feste Substrat aufgebracht, etwa durch Spritzen, Spachteln, Rakeln, Bürsten, Rollen oder Gießen. Die Beschichtungsstärke liegt in der Regel im Bereich von 3 bis 500 $g/m^2$ und insbesondere 10 bis 200 $g/m^2$ bzw. Naßfilmdicken von etwa 3 bis 500 $\mu m$ und insbesondere 50 bis 200 $\mu m$. Das Aufbringen kann dabei sowohl bei Raumtemperatur als auch bei erhöhter Temperatur, insbesondere jedoch nicht oberhalb von 100 °C erfolgen.

**Beispiele**

**A) Meßmethoden**

**Shore-D-Härte:** Die Bestimmung der Shore-D-Härte erfolgte gemäß DIN 53505 bei 23 °C.
Wie oben beschrieben wurden die Substanzen, deren Shore-D-Härte gemessen wurde, durch radikalisch initiierte Härtung (durchgeführt in Gegenwart von 5 Gew.-% - bezogen auf die Substanz - tert.-Butylperoxidbenzoat bei 160 °C in einem Zeitraum von 30 Minuten) der erfindungsgemäßen Fett(meth)acrylate hergestellt.

**%EpO:** Zur Charakterisierung des Gehalts von Verbindungen an Oxirangruppen ("Epoxidgruppen") wurde eine Epoxidtitration durchgeführt. Die dabei erhaltene **Epoxidzahl (%EpO)** gibt an, wieviel Gramm Oxiran-Sauerstoff in 100 Gramm einer Probe enthalten sind.
Der Titration liegt folgendes Prinzip zu Grunde: Man fügt eine Lösung mit überschüssigem Tetraethylammoniumbromid zur Probe, die Oxiranringe enthält. Danach titriert man die Mischung mit einer Lösung von Perchlorsäure in Eisessig, wobei eine äquimolare Menge Bromwasserstoff freigesetzt wird. Der Bromwasserstoff reagiert unter Ringöffnung mit den Oxiranringen und bildet das entsprechende Bromhydrin.

$$((CH_3\text{-}CH_2)_4N)^{\oplus}\,Br^{\ominus} + HClO_4 \rightarrow ((CH_3\text{-}CH_2)_4N)^{\oplus}ClO_4^{\ominus} + HBr$$

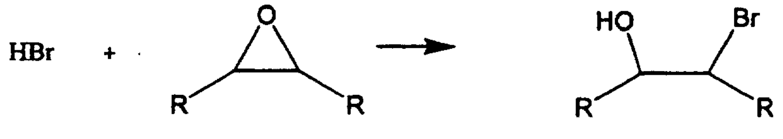

Als Indikator setzt man Kristallviolett ein. Die Bestimmung setzt die Abwesenheit von Wasser, Basen und Aminen voraus.
Es kamen folgende Reagentien zum Einsatz:(1) 0,1-N-Perchlorsäure (Fa. Merck) in Eisessig; (2)

Tetraethylammoniumbromid (Fa. Fluka) in Form einer Lösung von 100 g Tetraethylammoniumbromid in 400 ml Eisessig; (3) Kristallviolett (Fa. Merck); zur Herstellung der Indikatorlösung wurden 0,2 g Kristallviolett in 100 ml Eisessig gelöst.
Durchführung:0,2 bis 0,5 g der Probe, die Oxiranringe enthält werden in einem Erlenmeyerkolben vorgelegt. Die Probe wird in 50 ml wasserfreiem Aceton gelöst. Dann fügt man 10 ml Tetraethylammoniumbromidlösung (siehe oben) und 3 Tropfen Kristallviolettlösung (siehe oben) hinzu. Die Mischung wird mit einer 0,1-N Lösung von Perchlorsäure in eisessig titriert. Der Endpunkt ist erreicht, sobald die Farbe sich von blau nach grün ändert.
Vor Durchführung der eigentlichen Titration führt man eine Blindprobe durch (diese enthält keine Oxiranverbindung), um Meßfehler auszuschließen.
Auswertung:Der Epoxidgehalt %EpO wird wie folgt berechnet:

$$\% \, EpO = [(a - b) * 0{,}160] / E$$

a : = Milliliter 0,1 n $HClO_4$ Lösung, die zur Titration benötigt wird
b : = Milliliter 0,1 n $HClO_4$ Lösung, die im Blindversuch benötigt wird
E : = Einwaage der Probe in Gramm

**B) Herstellung und Prüfung von Fettacrylaten**

Tabelle1:

| Kennzahlen der eingesetzten Öle | | | | | |
|---|---|---|---|---|---|
| | | Fettsäurespektrum* | | | |
| | ***Jodzahl*** | *16:0 + 18:0* | *18:1* | *18:2* | *18:3* |
| Sojaöl | 130 | 19 | 21 | 53 | 7 |
| Leinöl | 180 | 10 | 17 | 14 | 59 |
| Hanföl | 162 | 9 | 12 | 56 | 21 |
| Linolaöl | 140 | 10 | 16 | 72 | 2 |

* wie dem Fachmann bekannt gibt die Zahl vor dem Doppelpunkt die Kettenlänge an (Anzahl der C-Atome), die Zahl hinter dem Doppelpunkt die Anzahl der C=C-Doppelbindungen.

Um gute Materialeigenschaften zu erhalten wurden epoxidierte Fette und Öle mit dem maximal erreichbaren Oxirangehalt verwendet.
Im Falle von epoxidiertem Leinöl und Hanföl betrug dieser ca. 8,2 %EpO, da bei höheren Gehalten eine Verfestigung eintritt. Für epoxidiertes Linolaöl betrug der Epoxidgehalt ca. 7,1 %EpO und für epoxidiertes Sojaöl ca. 6,5 % EpO.

**Vergleichsbeispiel 1: Sojaölacrylat (V 1)**

Zu einer Mischung von 1636 g Sojaölepoxid (% EpO = 6,3), 30 g Triethylamin und 6000 ppm Hydrochinonmethylether (MEHQ) wurden im Verlauf von 10 Stunden bei 130 °C 465 g Acrylsäure unter kräftigem Rühren und Einleiten von Sauerstoff zugetropft. Die Reaktion wurde beendet, als die Säurezahl weniger als 50 betrug.
Das Produkt wies einen % EpO-Wert von 0,4 auf.

**Vergleichsbeispiel 2: Leinölacrylat (V 2)**

Zu einer Mischung von 1000 g Leinölepoxid (% EpO = 8,5), 30 g Triethylamin und 6000 ppm MEHQ wurden im Verlauf von 10 Stunden bei 130 °C 394 g Acrylsäure unter kräftigem Rühren und Einleiten von Sauerstoff zugetropft. Die Reaktion wurde beendet als die Säurezahl weniger als 50 betrug.
Das Produkt wies einen % EpO-Wert von 0,5 auf

**Beispiel 1: Linolaölacrylat (B1)**

Zu einer Mischung von 1195 g Linolaölepoxid (Epoxidzahl = 7,1), 30 g Triethylamin und 6000 ppm MEHQ wurden im Verlauf von 10 h bei 130 °C 382 g Acrylsäure unter krüftigem Rühren und Einleiten von Sauerstoff zugetropft Die Reaktion wurde beendet als die Säurezahl weniger als 50 betrug.
Das Produkt wies einen % EpO-Wert von 0,4 auf.

**Anwendungstechnische Untersuchungen:**

Als Maß für die im ausgehärteten Polymer erreichbaren Eigenschaften der Fettacrylate wurden diese zusammen mit 5 Gewichtsprozent tert-Butylperoxybenzoat 30 min bei 160 °C ausgehärtet. Die Ergebnisse sind in Tabelle 2 zusammengefaßt.

Aus Tabelle 2 geht klar hervor, daß die beiden in der Aufgabenstellung formulierten Bedingungen, nämlich die Bereitstellung gießbarer härtbarer Substanzen, die einerseits eine Viskosität von weniger als 30.000 mPas und vorzugsweise von weniger als 20.000 mPas (gemessen in Substanz nach Brookfield bei 23 °C) aufweisen und gleichzeitig sich dadurch auszeichnen sollten, daß sie bei der radikalisch initiierten Härtung (durchgeführt in Gegenwart von 5 Gew.-% - bezogen auf die Substanz - tert.-Butylperoxidbenzoat bei 160 °C in einem Zeitraum von 30 Minuten) zu einem Polymeren mit einer Shore-D-Härte (bestimmt bei 23 °C nach DIN 53505) von mindestens 60 führen, nur vom erfindungsgemäßen Beispiel B 1 erreicht wird, nicht jedoch von den Vergleichsbeispielen.

Tabelle 2:

| | Viskosität des Fettacrylats [mPas] | Shore-D- Härte des ausgehärteten Polymers |
|---|---|---|
| Sojaölacrylat (V 1) | 14.000 | < 50 |
| Leinölacrylat (V 2) | 210.000 | 80 |
| **Linolaölacrylat (B 1)** | 19.000 | 70 |

**Patentansprüche**

1. Fett(meth)acrylate, erhältlich durch Umsetzung von epoxidierten Fettsäurestern und/oder epoxidierten Triglyceriden mit Acrylsäure und/oder Methacrylsäure, wobei die Umsetzung so geführt wird, daß der %-EpO-Gehalt der Fett(meth)acrylate unterhalb von 0,6 liegt und wobei für die den Fett(meth)acrylaten zu Grunde liegenden epoxidierten Fettsäurester und/oder epoxidierten Triglyceride folgende Maßgaben gelten:

    a) Der %-EpO-Gehalt der eingesetzten epoxidierten Fettsäurestern und/oder epoxidierten Triglyceride liegt oberhalb von 6,4.
    b) Weniger als 20 mol-% der den epoxidierten Fettsäurestern und/oder epoxidierten Triglyceriden zu Grunde liegenden Fettsäurebausteine enthalten 3 oder mehr C=C-Doppelbindungen pro Fettsäurebaustein.
    c) Mehr als 60 mol-% der den epoxidierten Fettsäurestern und/oder epoxidierten Triglyceriden zu Grunde liegenden Fettsäurebausteine enthalten 2 C=C-Doppelbindungen pro Fettsäurebaustein.

2. Verwendung von Fett(meth)acrylaten nach Anspruch 1 zur Herstellung von Beschichtungsmassen.

3. Gießbare, härtbare Zusammensetzungen mit einem Gehalt von einem oder mehreren Fett(meth)acrylaten nach Anspruch 1.

4. Zusammensetzungen nach Anspruch 3, wobei zusätzlich mit den Fett(meth)acrylaten radikalisch copolymerisierbare Verbindungen enthalten sind.

**Claims**

1. Fatty (meth)acrylates obtainable by reaction of epoxidized fatty acid esters and/or epoxidized triglycerides with acrylic acid and/or methacrylic acid, the reaction being carried out in such a way that the %-epo content of the fatty (meth)acrylates is below 0.6 and the following provisos applying to the epoxidized fatty acid esters and/or epoxidized triglycerides on which the fatty (meth)acrylates are based:

a) the %-epo content of the epoxidized fatty acid esters and/or epoxidized triglycerides used is above 6.4,
b) less than 20 mol-% of the fatty acid units on which the epoxidized fatty acid esters and/or epoxidized triglycerides are based contain 3 or more C=C double bonds per fatty acid unit,
c) more than 60 mol-% of the fatty acid units on which the epoxidized fatty acid esters and/or epoxidized triglycerides are based contain 2 C=C double bonds per fatty acid unit.

**2.** The use of the fatty (meth)acrylates claimed in claim 1 for the production of coating compositions.

**3.** Curable casting compositions containing one or more of the fatty (meth)acrylates claimed in claim 1.

**4.** Compositions as claimed in claim 3 additionally containing compounds radically copolymerizable with the fatty (meth)acrylates.

## Revendications

**1.** (Méth)acrylates gras pouvant être obtenus par réaction d'esters d'acide gras époxydés et/ou de triglycérides époxydés, avec de l'acide acrylique et/ou de l'acide méthacrylique, la réaction étant effectuée de telle sorte que la teneur en % d'EpO des (méth)acrylates gras se situe en dessous de 0,6, et les esters d'acide gras époxydés et/ou les triglycérides époxydés à la base des (méth)acrylates gras ayant les caractéristiques suivantes :

a) la teneur en % d'EpO des esters d'acide gras époxydés et/ou des triglycérides époxydés mis en oeuvre, se situe au-dessus de 6,4,
b) moins de 20 % molaire des éléments d'acides gras à la base des esters d'acide gras époxydés et/ou des triglycérides époxydés renferment au moins 3 doubles-liaisons C = C par élément d'acide gras,
c) plus de 60 % molaire des éléments constitutifs d'acide gras à la base des esters d'acide gras époxydés et/ou les triglycérides époxydés, renferment deux double-liaisons C = C par élément d'acide gras.

**2.** Utilisation de (méth)acrylates gras selon la revendication 1, pour la production de masses de revêtement.

**3.** Compositions coulables, durcissables, renfermant un ou plusieurs (méth)acrylates gras selon la revendication 1.

**4.** Compositions selon la revendication 3, renfermant en plus des (méth)acrylates gras, des composés copolymérisables par voie radicalaire.